# EUROPÄISCHE PATENTSCHRIFT

(11) **EP 0 720 479 B1**
(45) Veröffentlichungstag und Bekanntmachung des Hinweises auf die Patenterteilung: **02.07.2003**
(21) Anmeldenummer: 94928367.5
(22) Anmeldetag: 16.09.1994
(51) Int. Cl.: A61K 31/616, A61K 9/70, A61P 29/00

(54) **TRANSDERMALES THERAPEUTISCHES SYSTEM MIT DEM WIRKSTOFF ACETYLSALICYLSÄURE**
TRANSDERMAL THERAPEUTIC SYSTEM WITH ACETYLSALICYLIC ACID AS ACTIVE SUBSTANCE
SYSTEME THERAPEUTIQUE TRANSDERMIQUE CONTENANT COMME PRINCIPE ACTIF L'ACIDE ACETYLSALICYLIQUE

(30) Priorität: 22.09.1993 DE 4332093
(43) Veröffentlichungstag der Anmeldung: 10.07.1996
(73) Patentinhaber: LTS Lohmann Therapie-Systeme AG, 56626 Andernach (DE)
(72) Erfinder: HORSTMANN, Michael, D-56564 Neuwied (DE); HOFFMANN, Gerd, D-56626 Andernach (DE); KINDEL, Heinrich, D-56581 Ehlscheid (DE)
(74) Vertreter: Flaccus, Rolf-Dieter, Dr.
(86) Internationale Anmeldenummer: EP9403106
(87) Internationale Veröffentlichungsnummer: WO95008330

(56) Entgegenhaltungen:
- EP-A- 0 156 080
- WO-A-88/10111
- WO-A-92/14442
- DE-A- 4 241 128

## Beschreibung

Die Erfindung betrifft ein transdermales therapeutisches System zur Abgabe von Acetylsalicylsäure und gegebenenfalls weiterer Stoffe über die Haut an den menschlichen Körper. Das beschriebene System zeigt erhöhte chemische Hydrolysestabilität für den Wirkstoff Acetylsalicylsäure.

Transdermale therapeutische Systeme (TTS) sind in der arzneilichen Therapie einer Reihe von Erkrankungen im Markt eingeführt.

Auch die grundsätzliche Permeationsfähigkeit des Wirkstoffes Acetylsalicylsäure durch die menschliche Haut und damit die Eignung als Bestandteil von transdermalen therapeutischen Systemen ist bekannt. So beschreiben Rougier et al. (J. Pharm. Sci. 176, 451-454, 1987) bereits die (eher geringe) Abhängigkeit der Acetylsalicylsäure-Aufnahmerate in die Haut vom ausgewählten Hautareal.
Chen et al. (Zhongguo Yiyuan Yaoxue Zazhi Bd. 11, S. 245-247 (1991)) berichten über einen erfolgreichen transdermalen Einsatz von Acetylsalicylsäure-Cremes bei rheumatischen Kindern.

In JP 3 112 926 wird ein Mittel zur perkutanen Applikation, auch von Acetylsalicylsäure, beschrieben, welches durch Auflösen in Wasser und Zugabe wasserquellender Zusatzstoffe entsteht, die schließlich in einem Siliconpolymer dispergiert zum Einsatz kommen.
Hier besteht wegen des Kontaktes zu Wasser große Hydrolysegefahr.

Acetylsalicylsäure ist ein therapeutisch gut und seit langem eingeführter Arzneistoff mit einem hohen therapeutischen Index. In sehr hohen Dosen (über ein Gramm pro Tag) ist sie als Antirheumatikum in Gebrauch, in mittleren Dosen (250 bis 500 mg) als Antipyretikum/Analgetikum und in niedriger Dosierung (30 bis 150 mg pro Tag) als Thrombozytenaggregationshemmer.
Acetylsalicylsäure schmilzt bereits bei niedriger Temperatur (ca. 139°C) und ist bei dieser Temperatur bereits merklich flüchtig.
Acetylsalicylsäure kommt in mehreren polymorphen Formen (Modifikationen) vor, die zum Teil bereits bei 100°C schmelzen und auch ein unterschiedliches Auflösungsverhalten zeigen.

Wegen der instabilen Estergruppierung ist sie anfällig für Hydrolyse und Umesterung. Auf dem Wege zu einer stabilen Arzneiform sind daher generell folgende Regeln zu beachten:
- Wasser, Alkohole und Ester sind als mögliche Reaktionspartner in der Regel ungeeignete Rezepturbestandteile.
- Aus dem gleichen Grunde ist unter Beachtung des Massenwirkungsgesetzes der Zusatz von potentiellen Reaktionsprodukten, wie Essigsäure oder wasserbindendem Acetanhydrid als stabilisierenden Komponenten sinnvoll.
- Da die Hydrolyse bei ca. pH 2 bis 3 am langsamsten läuft, ist eine Einstellung auf diesen Säuregrad stabilitätsfördernd.
- Auch der Einsatz weiterer stabilisierender Hilfsstoffe ist bekannt, zum Beispiel empfehlen Luzzi und Ma (US-PS 4,228,162) Dimethylisosorbid als stabilisierendes Lösungsmittel.

In der Literatur wurden keine Angaben gefunden, wie diese galenische Problematik auf die Technik zur Herstellung von TTS anzuwenden ist. Erste Formulierungen, bei denen die TTS-Matrix, dem Stand der Technik folgend, Acetylsalicylsäure ausschließlich in gelöster Form enthielt, erwiesen sich als zu instabil.

Nach der US-PS 4,286,592 wird ein pharmazeutischer Wirkstoff in kristalliner Form in einem TTS eingesetzt, um die Wirkstoffabgabe über eine Klebschicht zu steuern. Maßgeblich ist dabei ein spezielles Verhältnis zwischen Korngröße der Kristalle und Diffusionseigenschaften.
Diese Anwendung würde der Fachmann jedoch nur zur Limitierung eines Wirkstoffflusses einsetzen, nicht aber zur Stabilisierung bei gleichzeitigem Erhalt der maximal möglichen Freisetzungseigenschaften.

Es ist zwar gelegentlich übliche Praxis, transdermale therapeutische System mit so hohen Konzentrationen an Wirkstoff zu beladen, daß dieser nach anfänglich vollständiger Lösung während der Herstellung auskristallisiert (EP 0 156 080). Hierdurch wird eine besonders lang anhaltende Wirkung erreicht. Das Verfahren hat jedoch den Nachteil, daß die Art und Weise der Kristallisation schwer zu kontrollieren ist und daß ohne Anwesenheit von Kristallisationskeimen sehr häufig nur begrenzt haltbare Modifikationen erhalten werden. Auf diese Weise ist das Produkt nicht in der erforderlichen pharmazeutischen Qualität herstellbar. Hinweise auf den Aspekt der Stabilisierung von Wirkstoffen durch Einbringung eines hohen kristallinen Anteils in transdermale therapeutische Systeme sind der Literatur nicht zu entnehmen.

Aufgabe der Erfindung ist die Bereitstellung eines transdermalen therapeutischen Systems mit Acetylsalicylsäure, welches eine ausreichende pharmazeutische Stabilität bei unter diesen Umständen maximal erreichbarer Abgabegeschwindigkeit an die Haut aufweist.

Diese Aufgabe wird erfindungsgemäß gelöst durch ein transdermales therapeutisches System, enthaltend den Wirkstoff Acetylsalicylsäure, mit geschichtetem Aufbau aus einer im wesentlichen wirkstoff- und feuchtigkeitsundurchlässigen Rückschicht sowie einer oder mehrerer wirkstoffhaltiger Matrixschichten, von welchen mindestens eine der Matrixschichten Acetylsalicylsäure zum überwiegenden Teil in kristalliner Form enthält, wobei das besagte transdermale therapeutische System durch seine Herstellung gekennzeichnet ist, bei welcher Acetylsalicylsäure in einer Suspension bzw. in Lösung oder Schmelze des Matrix-Grundmaterials aus Acrylsäureester enthaltenden Copolymeren, Mischungen aus Kautschuken und Harzen sowie Polyvinylacetat auf einem folienförmigen, dehäsiv ausgerüsteten Grundmaterial in einer Schicht aufgebracht und getrocknet wird, worauf die Schicht durch Auflegen einer im wesentlichen wirkstoff- und feuchtigkeitsundurchlässigen Rückschicht abgedeckt und das Substrat durch Konturstanzung bzw. Folienschnitt vereinzelt und vereinzelte TTS in gasdicht versiegelte Packmittel verpackt werden, wobei das Packmittel durch wasseraufnehmende Ausrüstung dem transdermalen therapeutischen System Feuchtigkeit entzieht.

Bei Wirkstoffkristallen ist gewöhnlich eine unerwünschte Limitierung der Freisetzungseigenschaften dadurch zu befürchten, daß die Auflösungsgeschwindigkeit kleiner werden könnte als die Permeation in die Haut. Dies ist jedoch überraschenderweise nicht der Fall, wenn erfindungsgemäß außer durch sorgfältige Trocknung der schichtförmigen Bestandteile des transdermalen therapeutischen Systems oder sonstige bekannte Stabilisierungsmaßnahmen bereits vor der Beschichtung und Trocknung eine stabilisierende Phase aus kristalliner Acetylsalicylsäure erzeugt wird. Selbstverständlich sind zur Stabilisierung dieser Eigenschaften trockene Lagerbedingungen sehr hilfreich, da so die Hydrolyse zurückgedrängt wird. Solche Lagerbedingungen lassen sich durch gas- und feuchtigkeitsdichte Packmittel sowie durch Einlegen von einem oder mehreren feuchtigkeitsabsorbierenden Körpern in das Packmittel gewährleisten, wie dies bei den erfindungsgemäßen transdermalen therapeutischen Systemen nach Anspruch 1 vorgesehen ist.

Der Erfindungsgegenstand wird im folgenden näher beschrieben:

Wesentlich für die vorliegende Erfindung ist die Anwesenheit von ungelöster Acetylsalicylsäure in einem transdermalen therapeutischen System. Im Gegensatz zum Stand der Technik wird dabei zumindest eine Teilmenge des vor dem Trocknungsprozeß eingesetzten Wirkstoffes während der gesamten Fertigung in ungelöstem Zustand gehalten, damit zumindest genügend Impfkristalle der stabilen Wirkstoffmodifikation in das Produkt gelangen. So wird ein nur vorübergehend stabiler, übersättigter Zustand der Lösung des Wirkstoffes - unter Umständen unter Fällung von metastabilen Niederschlägen - vermieden, der große Stabilitätsrisiken bergen würde. Im Laufe der Zeit führen unkontrollierte Rekristallisationen zu einem nicht mehr der Anfangsspezifikatione der Freisetzungsgeschwindigkeit entsprechenden Produkt.

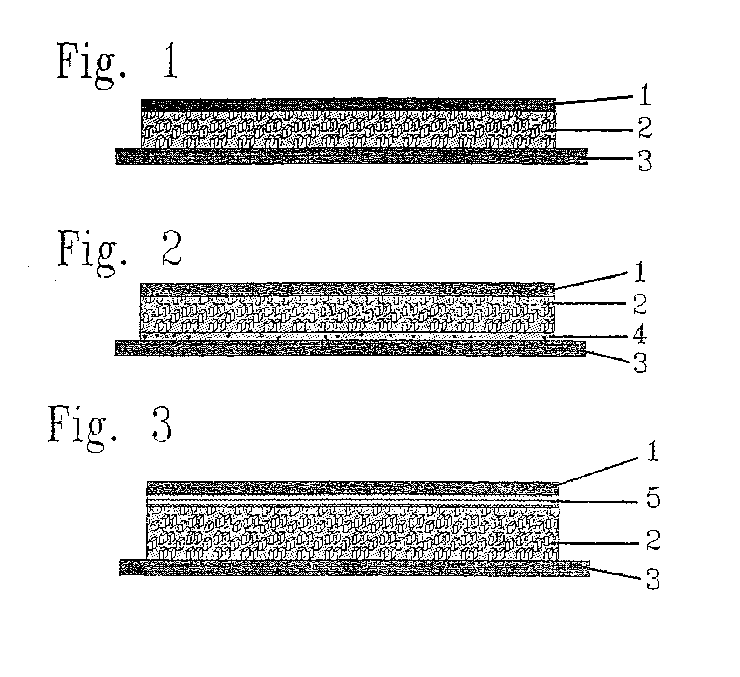

Die individuelle Ausgestaltung der Erfindung kann sehr unterschiedlich sein. Im einfachsten Falle entspricht das System nach Fig. 1 einer einschichtigen Matrix mit selbstklebenden Eigenschaften auf der Haut. Das System besteht aus einer im wesentlichen wirkstoffundurchlässigen Rückschicht, der Matrixschicht sowie einer vor Anwendung zu entfernenden ablösbaren Schutzfolie.

Als Rückschicht (1) eignen sich zahlreiche Einsatzstoffe, unter diesen Polyester, die durch besondere Festigkeit und Diffusionsfestigkeit ausgezeichnet sind, aber auch nahezu beliebige andere zur Verwendung auf der Haut geeignete Kunststoffe, wie Polyvinylchlorid, Ethylenvinylacetat, Vinylacetat, Polyethylen, Polypropylen, Cellulosederivate und viele andere mehr. Im Einzelfall kann ein zusätzliche Auflage, z.B. durch Bedampfung von Metallen oder anderen diffusionssperrenden Zusatzstoffen wie Siliciumdioxid, Aluminiumoxid o.ä. vorgenommen werden. Auch wird die Rückschicht zur verbesserten Akzeptanz häufig auf der Außenseite hautfarben lackiert, oder auf andere Weise behandelt, um ein verbessertes Erscheinungsbild zu erreichen.
Die Dicke der folienartigen Rückschicht beträgt gewöhnlich, abhängig u.a. von der Festigkeit und Durchlässigkeit des gewählten Materials, 8 bis 80 µm; sie kann jedoch für spezielle Zwecke auch dicker oder dünner eingestellt werden.

Für die Matrixschicht (2) des erfindungsgemäßen Pflasters kommen Acrylsäureester enthaltende Copolymere, Mischungen aus Kautschuken und Harzen sowie Polyvinylacetat in Frage. Der Zusatz von bis zu 40 % Füllstoffen wie Titandioxid, Zinkdioxid, Kreide, Aktivkohle, feinverteiltes Siliciumdioxid etc. behindert keineswegs die erfindungsgemäße Funktion und kann Vorteile hinsichtlich der Kohäsion des gefertigten Systems ergeben.

Weniger bedeutsam für die Funktion ist die wiederablösbare Schutzschicht (3), welche zum Beispiel auch aus einem Polyestermaterial bestehen kann, aber auch aus beliebigen anderen, zur Verwendung auf der Haut geeigneten Kunststoffen, wie Polyvinylchlorid, Ethylenvinylacetat, Vinylacetat, Polyethylen, Polypropylen, Cellulosederivate oder vielen anderen mehr. Im Einzelfall kann Bedampfung mit Metallen oder anderen diffusionssperrenden Zusatzstoffen wie Siliciumdioxid, Aluminiumoxid o.ä. vorgenommen werden. In jedem Fall ist zur Seite der klebenden Matrix hin eine Oberflächenbeschichtung mit dehäsiven Materialien, zum Beispiel Siliconen oder fluorhaltigen Kunststoffen erforderlich, damit der Verbund leicht ablösbar bleibt. Die Dicke der folienartigen wiederablösbaren Schutzschicht beträgt gewöhnlich ca. 40 bis 200 µm; sie kann für spezielle Zwecke auch dicker oder dünner eingestellt werden.

In Fig. 2 ist als weitere erfindungsgemäße Ausgestaltung die gleichfalls mögliche Aufteilung in zwei Matrixschichten sichtbar, wobei vorteilhafterweise die Klebschicht (4) kleinere Wirkstoffkristalle und diese in geringer Häufigkeit enthält. So kann eine bessere Hauthaftung erreicht werden als mit einem einschichtigen System. Ein vollständiger Verzicht auf kristalline Anteile ist auch in der Klebschicht für die gestellte Aufgabe nicht zweckmäßig, da das Risiko des Entstehens einer zu breiten Diffusionsgrenzschicht besteht, welche geringeren Fluß in die Haut zur Folge hätte.

Die Größe der Acetylsalicylsäure-Kristalle ist für die Funktion des erfindungsgemäßen Prinzips und des grundsätzlichen Vorteils der Stabilisierung an sich ohne Bedeutung. Vorteilhaft sind sicher schon wegen des homogeneren optischen Eindrucks kleinere Kristalle unter ca. 50 bis 100 µm Durchmesser. Bei Verwendung extrem schlecht lösender Matrix-Grundmaterialien, wie zum Beispiel Massen auf Basis von Polyisobutylen und größeren Kristalldurchmessern (über 300 µm) kann eine Freisetzungskontrolle des Wirkstoffes durch die Haut eintreten mit dem Vorteil eine systemseitigen Kontrolle der Freisetzungsgeschwindigkeit aber dem Nachteil, daß nur ein Teil der theoretisch erreichbaren Flußrate erzielt werden kann.

Unabhängig vom Aufbau des Systems können flüssige Zusatzstoffe zugesetzt werden wie z.B. 2-Pyrrolidon, Benzylalkohol, Butanol und andere kurzkettige Alkohole, Triglyceride, Cholesterol, Cineol, delta-Tocopherol, Diethylenglycol, Diethylenglycolmonoethylether, Diisopropyladipat, Dimethyldecylphosphoxid, Dimethylisosorbid, Dimethyllauroylamid, Dimethylsulfoxid, Dodecylsulfoxid, Essigsäure, Ethylacetat und andere aliphatische und aromatische Ester, Ethylenglycol, Ethylenglycolmonolaurat und andere Ester und Ether von Ethylenglycol oder Propylenglycol, 2-Octyldodecanol, dünnflüssiges Paraffin, Glycerin, Glycerinmonooleat, Glycerinmonostearat, hydriertes Rizinusöl, Isopropylmyristat, Isopropylpalmitat, Laurinsäurediethanolamid, Menthol oder andere flüchtige Terpenderivate (die Gemischbestandteile vieler natürlicher etherischer Öle sind), Methylbenzoat, Methyloctylsulfoxid, Mono- oder Diethylacetamid, N,N-Diethyl-m-toluamid, N-Methylpyrrolidon, Octanol-1 und andere flüchtige mittelkettige Alkohole, Octansäure und andere mittelkettige aliphatische Carbonsäuren, Oleylalkohol, Olivenöl, Ölsäure, Ölsäureoleylester, Phenylethanol, Propylenglykol, Rizinolsäure, Triacetin, aber auch Mischungen solche Stoffe wie zum Beispiel Ölsäure/Propylenglykol.
Dabei ist jedoch im Einzelfall die Reaktionsfähigkeit des Wirkstoffes Acetylsalicylsäure mit Estern und Säuren sowie Alkoholen zu berücksichtigen, die den Einsatz solcher Stoffe limitiert. Die vorliegende Erfindung grenzt jedoch die mögliche chemische Reaktion durch Kompartimentierung weitestmöglich ein und bedeutet daher auch in dieser Hinsicht einen Stabilitätsvorteil.

Hierbei ist eine Variante besonders vorteilhaft, bei welcher die Matrixschicht in zwei aufeinander zu laminierende Anteile aufgeteilt wird, die beide erfindungsgemäß Acetylsalicylsäure, überwiegend in kristalliner Form enthalten, von denen der eine Anteil einen im System verbleibenden leicht flüchtigen Inhaltsstoff (zum Beispiel einen der im vorstehenden Absatz genannten) als Lösemittel für die Restbestandteile enthält und mit dem zweiten Anteil der Matrixschicht bei der Herstellung laminiert wurde. So kann nach Wanderung der leicht flüchtigen Komponente ein ausreichend scherstabiles System enthaltend leicht flüchtige Zusatzstoffe entstehen.

Der Gehalt der gesamten Matrix des erfindungsgemäßen transdermalen therapeutschen Systems an Acetylsalicylsäure liegt zweckmäßig bei etwa 15 bis 60 Gew.-%, bevorzugt bei 35 bis 50 Gew.-%. Besonders bei gut lösenden Matrixgrundlagen wie Acrylsäureestercopolymeren ist eine Überschreitung der Sättigungslöslichkeit um den Faktor 2 oder sogar etwa 5 bis 10 nur mit einer solchen sehr hohen Beladung des Systems mit Wirkstoff zu erreichen.

Es ist für das System nützlich, wenn nicht nur die erfindungsgemäße Stabilisierung gegen chemischen Abbau des Wirkstoffes, sondern auch eine Stabilisierung gegen physikalische Alterungseinflüsse erfolgt.

Eine solche ist mit dem hier beschriebenen Aufbau leicht möglich, indem man zur Herstellung die thermodynamisch stabilste Form der Acetylsalicylsäure, nach heutigem Kenntnisstand diejenige, welche einen Schmelzpunkt von oberhalb 132°C (abhängig vom Reinheitgrad ca. 139°C) aufweist, einsetzt und bei der Herstellung dafür sorgt, daß stets zumindest ein kleiner Teil des Wirkstoffes, homogen verteilt, in dieser ursprünglichen Kristallmodifikation bleibt. Im Extremfall genügt es, eine gesättigte Lösung von Acetylsalicylsäure und den pharmazeutischen Hilfsstoffen der Matrixgrundlage anzufertigen und in dieser nur etwa 1% der Acetylsalicylsäure in stabiler Modifikation kristallin zu dispergieren, diese Masse zu trocknen und mit weiteren Folien und/oder Matrixbestandteilen zu vereinigen.

Es ist dabei ebenfalls möglich, außer lösemittelbasierten Herstellungsverfahren auch Heißschmelzverfahren zur Anfertigung der Matrixschicht oder -schichten einzusetzen, jedoch ist auch hier darauf zu achten, daß im Prozeß nie vollständige Lösung der Wirkstoffteilchen einsetzt.

Zusammenfassend gibt die vorliegende Erfindung ein TTS an, bei dem Acetylsalicylsäure in ungelöster Form vorliegt.
Die Erzeugung einer stabilisierenden Phase aus kristalliner Acetylsalicylsäure ermöglicht eine ausreichende Stabilität bei maximaler Abgabegeschwindigkeit an die Haut.

Beschreibung der Abbildungen:
Fig. 1: Erfindungsgemäßes System mit einschichtiger Matrix
   1-wirkstoffundurchlässige Rückschicht
   2-Matrix mit gelöstem und kristallinem Wirkstoffanteil
   3-Ablösbare Schutzschicht
Fig. 2: Erfindungsgemäßes System nach Anspruch 3
   1-Wirkstoffundurchlässige Rückschicht
   2-Matrix mit gelöstem und kristallinem Wirkstoffanteil
   3-Ablösbare Schutzschicht
   4-Matrixschicht ("Klebschicht") mit kleineren und weniger häufigen Acetylsalicylsäurekristallen
Fig. 3: Erfindungsgemäßes System nach Anspruch 4
   1-Wirkstoffundurchlässige Rückschicht
   2-Matrix mit gelöstem und kristallinem Wirkstoffanteil
   3-Ablösbare Schutzschicht
   5-Hautferne Matrixschicht mit Acetylsalicylsäure, bestehend aus einer Lösung der Matrixbestandteile in einem im TTS verbleibenden Lösemittel

## Patentansprüche

1. Transdermales therapeutisches System, enthaltend den Wirkstoff Acetylsalicylsäure, mit geschichtetem Aufbau aus einer im wesentlichen wirkstoff- und feuchtigkeitsundurchlässigen Rückschicht sowie einer oder mehrerer wirkstoffhaltiger Matrixschichten, von welchen mindestens eine der Matrixschichten Acetylsalicylsäure zum überwiegenden Teil in kristalliner Form enthält, **gekennzeichnet durch** dessen Herstellung, wobei Acetylsalicylsäure in einer Suspension bzw. in Lösung oder Schmelze des Matrix-Grundmaterials aus Acrylsäureester enthaltenden Copolymeren, Mischungen aus Kautschuken und Harzen sowie Polyvinylacetat auf einem folienförmigen, dehäsiv ausgerüsteten Grundmaterial in einer Schicht aufgebracht und getrocknet wird, worauf die Schicht **durch** Auflegen einer im wesentlichen wirkstoff- und feuchtigkeitsundurchlässigen Rückschicht abgedeckt und das Substrat **durch** Konturstanzung bzw. Folienschnitt vereinzelt und vereinzelte TTS in gasdicht versiegelte Packmittel verpackt werden, und daß das Packmittel **durch** wasseraufnehmende Ausrüstung dem transdermalen therapeutischen System Feuchtigkeit entzieht.

2. Transdermales therapeutisches System nach Anspruch 1, **dadurch gekennzeichnet, daß** zumindest eine Teilmenge des vor dem Herstellungsprozeß eingesetzten Wirkstoffes während der gesamten Fertigung in ungelöstem Zustand gehalten wird, so daß eine ausreichende Zahl von Impfkristallen der stabilen Wirkstoffmodifikation in das System gelangt.

3. Transdermales therapeutisches System nach Anspruch 1, **dadurch gekennzeichnet, daß** Acetylsalicylsaure verwendet wird, welche als stabile, wasserfreie, oberhalb 132°C schmelzende Modifikation kristallin vorliegt.

4. Transdermales therapeutisches System nach Anspruch 1, bestehend aus zwei Matrixschichten, von denen die hautseitig gelegene haftklebend ausgerüstet ist, **dadurch gekennzeichnet, daß** die für deven Herstellung verwendete hautrerne Matrixschicht wasserfreie Acetylsalicylsäure in kristalliner Form enthält, dagegen die zur Erhaltung einer vollflächigen Klebkraft verwendete Klebschich Vergleichsweise weniger Acetylsalicylsäurekristalle enthält.

5. Transdermales therapeutisches System nach Anspruch 4, **dadurch gekennzeichnet, daß** beide Matrixschichten wasserfreie Acetylsalicylsäure in kristalliner Form enthalten und daß die hautferne Matrixschicht das Produkt einer Lösung der Matrixbestandteile in einem dauerhaft im TTS verbleibenden Lösemittel ist.

6. Transdermales therapeutisches System nach einem oder mehreren der vorhergehenden Ansprüche, **dadurch gekennzeichnet, daß** der Anteil von Acetylsalicylsäure am Matrixmaterial 15 bis 60 Gew.-%, bevorzugt 35 bis 50 Gew.-% beträgt.

7. Transdermales therapeutisches System nach einem oder mehreren der vorhergehenden Ansprüche, **dadurch gekennzeichnet, daß** das Lösemittel Permeationsbeschleuniger enthält.

## Claims

1. A transdermal therapeutic system comprising the active substance acetylsalicylic acid, having a layered structure consisting of a backing layer which is substantially impermeable to active substances and moisture, and one or several active substance-containing matrix layers, at least one of said matrix layers comprising acetylsalicylic acid in mainly crystalline form, **characterized by** its production, whereby the acetylsalicylic acid, in a suspension or solution or a melt of the matrix base material of acrylic acid ester-containing copolymers, mixtures of rubbers and resins as well as polyvinyl acetate, is applied in one layer to a film-like base material which has been rendered dehesive and is then dried, whereupon the layer is covered by applying a backing layer which is substantially impermeable to active substances and moisture, and the substrate is separated by contour punching or film cutting, and the separated TTS are packed in packaging means sealed in a gas-tight manner, and the packing material withdraws moisture from the transdermal therapeutic system by means of water-absorbing means.

2. The transdermal therapeutic system according to claim 1, **characterized in that** at least a partial amount of the active substance used prior to the production process is maintained in undissolved condition during the whole fabrication so that a sufficient number of seed crystals of the stable active substance modification comes into the system.

3. The transdermal therapeutic system according to claim 1 **characterized in that** acetylsalicylic acid is used which is present as stable, anhydrous modification in crystalline form melting above 132°C.

4. The transdermal therapeutic system according to claim 1 consisting of two matrix layers, the matrix layer facing the skin being pressure sensitive adhesive,
**characterized in that** the matrix layer remote from the skin, which is used for the production thereof, comprises anhydrous acetylsalicylic acid in crystalline form, whereas the adhesive layer, which is used in order to maintain an adhesive power over the whole area, comprises comparatively fewer acetylsalicylic acid crystals.

5. The transdermal therapeutic system according to claim 4, **characterized in that** both matrix layers comprise anhydrous acetylsalicylic acid in crystalline form and that the matrix layer remote from the skin is the product of a solution of the matrix components in a solvent permanently remaining in the TTS.

6. The transdermal therapeutic system according to one or several of the preceding claims **characterized in that** the portion of acetylsalicylic acid in the matrix material amounts to 15 to 60%-wt., preferably 35 to 50%-wt.

7. The transdermal therapeutic system according to one or several of the preceding claims **characterized in that** the solvent comprises permeation enhancers.

## Revendications

1. Système thérapeutique transdermique contenant, à titre de principe actif, de l'acide acétylsalicylique, de structure stratifiée constituée par une couche dorsale essentiellement imperméable au principe actif et à l'humidité, ainsi que par une ou plusieurs couches matricielles contenant le principe actif, au moins une des couches matricielles contenant de l'acide acétylsalicylique principalement sous forme cristalline, **caractérisé par** sa fabrication dans laquelle on applique en une couche de l'acide acétylsalicylique dans une suspension, respectivement dans une solution ou dans une masse fondue de la matière de base matricielle constituée par des copolymères contenant de l'ester acrylique, par des mélanges de caoutchoucs et de résines, ainsi que par de l'acétate de polyvinyle sur une matière de base en forme de feuille mince rendue anti-adhésive, et on la sèche; ensuite, on recouvre la couche par application d'une couche dorsale essentiellement imperméable au principe actif et à l'humidité et on isole le substrat par estampage, respectivement par découpage de la feuille, et on emballe des TTS isolés dans des conditionnements scellés, étanches aux gaz, et en ce que le conditionnement, grâce à l'effet d'absorption d'eau qui lui a été conféré, extrait l'humidité du système thérapeutique transdermique.

2. Système thérapeutique transdermique contenant, selon la revendication 1, **caractérisé en ce qu'**au moins une quantité partielle du principe actif mis en oeuvre avant le processus de fabrication est maintenue à l'état non dissous au cours de l'ensemble de la fabrication, si bien qu'un nombre suffisant de germes de cristallisation de la modification stable du principe actif aboutissent dans le système.

3. Système thérapeutique transdermique selon la revendication 1, **caractérisé en ce qu'**on utilise de l'acide acétylsalicylique qui est présent à l'état cristallin sous la forme d'une modification stable, anhydre, dont le point de fusion est supérieur à 132°C.

4. Système thérapeutique transdermique selon la revendication 1, constitué par deux couches matricielles dont la première située côté peau a été rendue auto-adhésive, **caractérisé en ce que** la couche matricielle éloignée de la peau utilisée pour sa fabrication contient de l'acide acétylsalicylique anhydre sous forme cristalline, tandis que la couche adhésive utilisée pour obtenir la force d'adhérence s'exerçant sur toute la surface contient une quantité comparativement inférieure de cristaux d'acide acétylsalicylique.

5. Système transdermique thérapeutique selon la revendication 4, **caractérisé en ce que** les deux couches matricielles contiennent de l'acide acétylsalicylique anhydre sous forme cristalline et **en ce que** la couche matricielle éloignée de la peau représente le produit d'une solution des constituants de la matrice dans un solvant subsistant de manière durable dans le TTS.

6. Système transdermique thérapeutique selon une ou plusieurs des revendications précédentes, **caractérisé en ce que** la fraction d'acide acétylsalicylique dans la matière matricielle s'élève de 15 à 60% en poids, de préférence de 35 à 50% en poids.

7. Système transdermique thérapeutique selon une ou plusieurs des revendications précédentes, **caractérisé en ce que** le solvant contient un accélérateur de la perméation.
